# EUROPEAN PATENT SPECIFICATION

(11) **EP 2 481 431 B1**
(45) Date of publication and mention of the grant of the patent: **16.09.2015**
(21) Application number: 10845619.5
(22) Date of filing: 17.11.2010
(51) Int. Cl.: A61M 13/00

(54) **DEVICE FOR INSUFFLATING GAS AND COLLECTING EFFLUENTS FROM THE BODY CAVITIES OF AN INDIVIDUAL**
VORRICHTUNG ZUM EINBLASEN VON GAS UND ZUR SAMMLUNG VON EFFLUENZIEN AUS DEM KÖRPER EINES PATIENTEN
DISPOSITIF POUR L'INSUFFLATION DE GAZ ET LA COLLECTE D'EFFLUENTS DE CAVITÉS CORPORELLES D'UN INDIVIDU

(30) Priority: 18.11.2009 ES 200931022
(43) Date of publication of application: 01.08.2012
(73) Proprietor: Costovici, Nicolas Anthony, 17480 Roses (ES)
(72) Inventor: Costovici, Nicolas Anthony, 17480 Roses (ES)
(74) Representative: Gallego Jiménez, José Fernando
(86) International application number: PCT/ES2010/000465
(87) International publication number: WO 2011/098629

(56) References cited:
- WO-A1-2005/105198
- FR-A- 841 867
- US-A- 4 704 102
- US-A- 5 098 375
- US-A- 5 738 656
- US-A1- 2005 148 954
- US-A1- 2006 100 500
- US-A1- 2008 294 090
- US-B2- 7 361 170

## Description

### Object of the invention.

The present invention refers to a device for insufflating gas and collecting effluents from the body cavities of an individual, preferably a device for insufflating gas by rectal route for the distension of the intestines during the execution of an imaging exploration of said intestines.

### Background of the invention.

The present invention refers to a device that permits the introduction of gas, such as air or carbon dioxide, in the intestines of a patient so that these are distended, thus allowing these diagnostic methods to be practiced by imaging exploration.

During such an exploration, the individual usually discharges or spills effluents out of the rectal cavity which may contaminate the means used to inject the gas and the place where the exploration is being performed.

A device for reducing these problems is known comprising: a gas insufflation apparatus, a first duct for supplying gas fed by the insufflation apparatus into a body cavity of the individual; barriers inserted in the first duct to prevent the passage of effluents from the individual into the insufflation apparatus, a reservoir for collecting said effluents, a member insertable in an individual's cavity, attachable to the first duct and provided with an expandable annular element for sealing the perimeter of the individual's cavity, and means for inflating the expandable annular element when the insertable member is introduced into the body cavity of the individual.

In this manner, upon inserting the insertable member in the rectal cavity of the individual, the effluents produced during the exploration pass through the first duct and are collected in the reservoir. This device has several problems, the main one being that the first duct performs both the insufflation of the gas and the collection of effluents through the same channel, which contributes to a greater contamination of the insufflation device. Furthermore, the insufflation apparatus must bear the excess pressure that can be produced during the diagnosis process, which reverts in the fact that it should absorb gases coming from the cavity, thus being contaminated although the barriers are effective to the passage of liquids and solids.

Also, with these devices a total extraction of the effluents is not achieved, since, although the insufflation gas stream obstructs the collection of effluents flowing through the duct in the opposite direction, part of these effluents remaining in the rectal cavity. These effluents remaining in the rectal cavity distort the images obtained by the radiologist during the imaging exploration, considerably complicating the diagnosis.

Thus, the document of the PCT application WO2005105198 A1 for a "Manually operated insufflator", of the firm E-Z-EM INC, describes a manual device for distending a subject's abdominal cavity comprising, in one embodiment, an insufflation gas reservoir, an insertable member for its insertion in an abdominal cavity and a manually operated pump for introducing the gas from the reservoir through an intermediate duct communicating with the insertable member. This device has been designed to carry out diagnosis tests execution during practical learning exercise. Therefore its construction is simple to obtain a reduced cost and does not present certain safety precaution means used in other devices, or these are simpler. In this way, to regulate the gas pressure in the cavity it comprises a valve in the intermediate duct, just before the manually operated pump, for intuitive operation, which allows the discharge of overpressure. This valve does not prevent effluents and gaseous pollutants from coming out through it right where the doctor operates the pump, thus reducing the aseptic handling. In this document effluents coming out from the patient circulate through the same duct as the one used to introduce the gas in the patient, what complicates the exit of effluents.

U.S. patent publication US4704102 for "Method of Eliminating intra-abdominal infections" of the firm Geneco Inc. is also known, which describes a method for irrigating a body cavity with an irrigation liquid or solution, ensuring the asepsis of said cavity, such as the bladder or colon. For this purpose, it uses a device comprising a member insertable into said cavity with a balloon for its attachment and for plugging the entry to the cavity. This insertable member is connected by means of some taps or valves with an irrigation liquid reservoir, drainage means to a collecting reservoir of the drainage once the irrigation has been carried out. This device enables the entrance of the irrigation liquid and the exit of the liquid once carried out the irrigation to be done through parallel ducts within the insertable member, avoiding the contamination of the irrigation liquid, said liquid being maintained within the cavity for the desired time. This device is not intended for carrying out colonoscopies by blowing air or gas, since it does not have suitable means for continuous and simultaneous handling of the introduction of pressurized gas and collecting the effluent, required during the diagnosis procedure.

### Description of the invention.

The device for insufflating gas and collecting effluents from the body cavities of an individual, object of this invention, has technical features aimed at improving patient comfort and ensuring the cleanliness and hygiene of the gas insufflation device.

The device comprises: a gas insufflation apparatus, a first duct for supplying the gas fed by the insufflation apparatus toward the interior of a body cavity of the individual, barriers inserted in the first duct to prevent the passage of effluents from the individual into the insufflation apparatus, a reservoir for collecting said effluents, a member that can be inserted in the body cavity of the individual, attachable to the first duct and provided with an expandable annular element for sealing the cavity perimeter of the individual, and inflation means for inflating the expandable annular element when the insertable member is introduced into the body cavity of the individual. The first duct has a by-pass which is connected to a second gas discharge duct, said second gas discharge duct being provided with a safety valve for the automatic discharge of gas to the outside when the pressure of the gas contained in the first duct exceeds a predetermined value, said by-pass being in the section between the insufflation apparatus and the barriers that block the passage of the effluents into the insufflation apparatus. The insertable member comprises, at its rear end: a first opening for connecting the first duct with a first inner channel of the insertable member, said first inner channel being provided with an outlet opening arranged in an area next to the front end of the insertable member; a second opening for connecting an effluent discharge duct with a second inner channel of the insertable member, said second inner channel being provided with an outlet opening arranged in an area next to the front end of the insertable member, said effluent discharge duct being connected by its rear end to the effluent collecting reservoir; and a third opening for connecting the inflation means of the expandable annular element with a third inner channel of the insertable member, said third inner channel being provided with an outlet opening situated inside the expandable annular element.

According to the invention, the by-pass is connected to a second gas discharge duct which is provided with a safety valve for the automatic discharge of gas to the outside when the pressure of the gas contained in the first duct exceeds a predetermined value, said by-pass being in the section between the insufflation apparatus and the barriers that block the passage of the effluents into the insufflation apparatus.

This by-pass of gas to the outside permits the excess gas in the cavity to be discharged without entering into the insufflation apparatus, preventing its contamination by the return gas from the rectal cavity of the individual. It also allows for an easier control of the inner pressure of the cavity with the direct escape to the outside, keeping the volume and pressure in the cavity of the patient more constant.

Furthermore, in comparison with the device described in the patent WO2005105198, the second gas discharge duct of the invention allows for the exit of the gas to take place outside the working area of the medical personnel avoiding its contamination, and providing improved hygienic and sanitary conditions with respect to those devices and apparatuses in which the safety valve is mounted directly or adjacent to the first duct.

At the same time, the insertable member comprises at its rear end: a first opening for connecting the first duct with a first inner channel of the insertable member, said first inner channel being provided with an outlet opening arranged in an area next to the front end of the insertable member; a second opening for connecting an effluent discharge duct with a second inner channel of the insertable member, said second inner channel being provided with an outlet opening arranged in an area next to the front end of the insertable member, said effluent discharge duct being connected by its rear end to the effluent collecting reservoir; and a third opening for connecting the inflation means of the expandable annular element with a third inner channel of the insertable member, said third inner channel being provided with an outlet opening situated inside the expandable annular element.

This allows the insufflation gas supply to the individual's cavity to be made by a channel that is completely independent from the route or path for collecting the effluents, which are picked up by another independent channel, thereby reducing the possibility of contamination of the insufflation apparatus by the effluent collected.

The insufflation apparatus almost continuously blows insufflation gas and said gas flow to the patient prevents the liquids and effluents of the rectum and colon from entering the first duct.

Since the gas enters by a channel and the effluents are collected by another different channel, this device permits to obtain a better elimination of said effluents. This facilitates the work of the radiologist performing the diagnostic procedure as the image obtained is not distorted by said effluents that are properly collected.

In one embodiment, the first conduit presents in the section between the by-pass connecting the second gas discharge duct and the end connected to the insufflation apparatus, a second by-pass to which a catheter is connected for the introduction of gas in a cavity of the patient through the mouth or the nose. Thus, during a gastrointestinal diagnosis, it is possible to carry out the distension of the small intestine by means of said catheter with a single insufflation apparatus by balancing the inner pressure distending both intestines from its two ends.

In another embodiment the length of the effluent discharge duct, connected to the second opening of the insertable member is sufficient to collect the effluents produced. However, the effluent discharge duct is connected at its rear end to the effluent collecting reservoir in case they are abundant.

The second gas discharge duct may comprise scented barriers that avoid the return gases discharged to the outside from causing discomfort owing to their odour.

The gas insufflation apparatus may comprise means for warming the gas to be supplied. These gas warming means comprise a thermostat that regulates the warming of gas to a temperature of about 37.5 Celsius degrees, so that the gas introduced into the cavities of the individual is at a temperature corresponding to the body temperature, instead of the low temperature that decompressed gas from a bottle usually has, for example. This improves the comfort of the patient who is subjected to a less stressful situation.

At least one of the barriers arranged in the first duct may comprise antiviral or antibacterial materials, so that the gas is evacuated to the outside cleaner. Likewise, at least one of the barriers may comprise a hydrophobic filter that prevents said gas discharged to the outside from presenting any kind of moisture or contaminant associated with water, while increasing the safety and cleaning of the insufflation device.

It is envisaged that the device comprises clamping means, clamps or a lock arranged in the first duct before the barriers and the effluent discharge duct, so that when the diagnosis has been performed the shed of effluents is avoided when removing and disposing the means designed for single use.

### Description of figures.

In order to complement the description that is being carried out and with the purpose of facilitating the understanding of the characteristics of the invention, the present description is accompanied by a set of drawings wherein, by way of a nonlimiting example, the following has been represented:

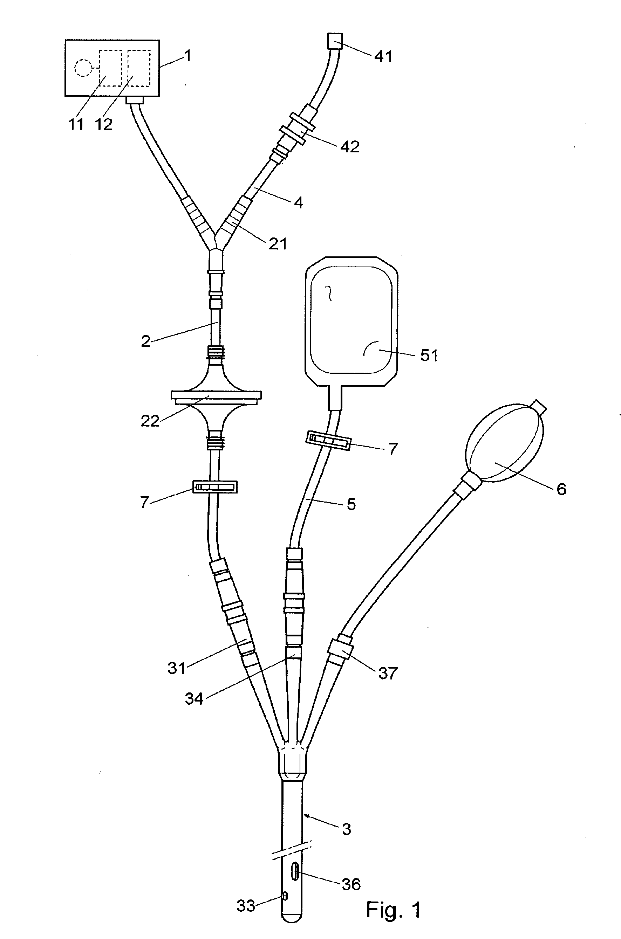

- Figure 1 shows a plan view of the device.

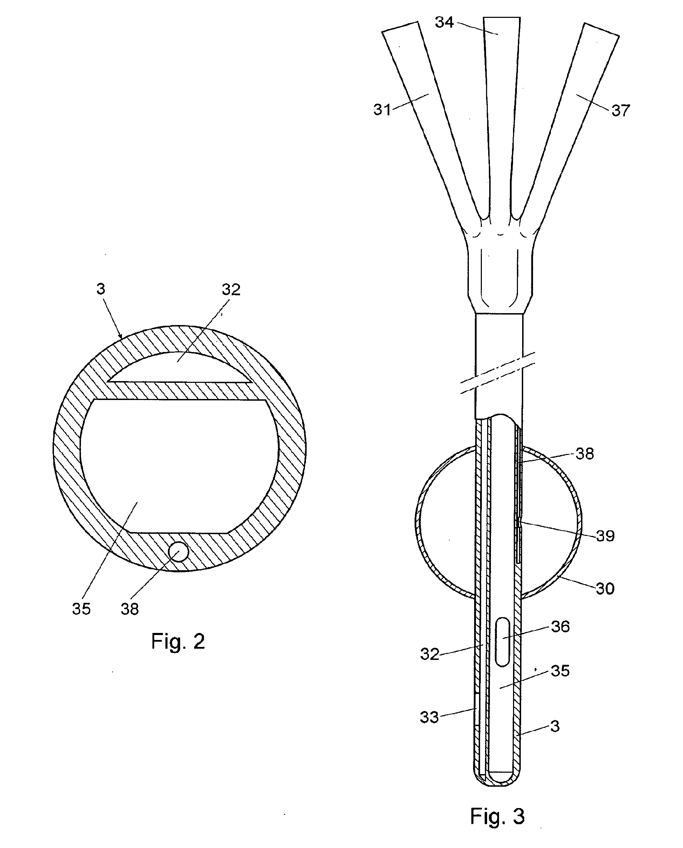

- Figure 2 shows a cross section of the insertable member.
- Figure 3 shows a longitudinal section of the insertable member.
- Figure 4 shows a plan view of the device in a configuration with the second catheter for its introduction through the nose or the mouth.

### Preferred embodiment of the invention.

As can be seen in the referenced figures the device for insufflating gas and collecting effluents from the body cavities of an individual, comprises a first gas insufflation apparatus (1) connected to a first duct (2) for supplying gas a cavity of an individual through an insertable member (3), the insufflation device (1) having gas heating means (11) and a thermostat (12) within regulated for supplying gas at 37.5 Celsius degrees approximately.

The first duct (2) comprises a bypass (21) near the end connected to the insufflation device (1), this bypass (21) being connected to a second duct (4) for the discharge of gas provided with a safety valve (41) for the automatic discharge of gas to the outside when the pressure of the gas contained in the first duct (2) exceeds a predetermined value. In said second duct (4) is a scented barrier (42) to prevent odours in the discharge of gases.

The first conduit (2) presents, after the by-pass (21), barriers (22) that comprise antiviral and antibacterial barriers and a hydrophobic filter.

At its rear end, the insertable member (3) comprises a first opening (31) for connecting the first duct (2), by its free end, with a first inner channel (32) provided with an outlet opening (33) arranged in an area next to the front end of said insertable member (3). The insertable member (3) also comprises a second opening (34) for connecting an effluent discharge duct (5) with a second inner channel (35) provided with an inlet opening (36) arranged in an area next to the front end of said insertable member (3). At the same time, the insertable member (3) comprises a third opening (37) for connecting inflation means (6) with a third inner channel (38), provided with an outlet opening (39) situated inside an expandable annular element (30), said expandable annular element (30) being situated around the insertable member (3) so as to block the entry of the cavity by means of its inflation. This insertable member (3) presents the front end rounded to facilitate its insertion in the rectal cavity without causing injury or discomfort.

The discharge duct (5) presents a length that is sufficient for storing the effluents for their disposal. At the end of this discharge duct (5) is an effluent collecting reservoir (51) of higher capacity.

In one embodiment, the first duct (2) and the effluent discharge duct (5) present coupled clamps (7) for their closing after use, preventing effluent spillages and possible contamination upon removing the insertable member (3) from the cavity of the individual.

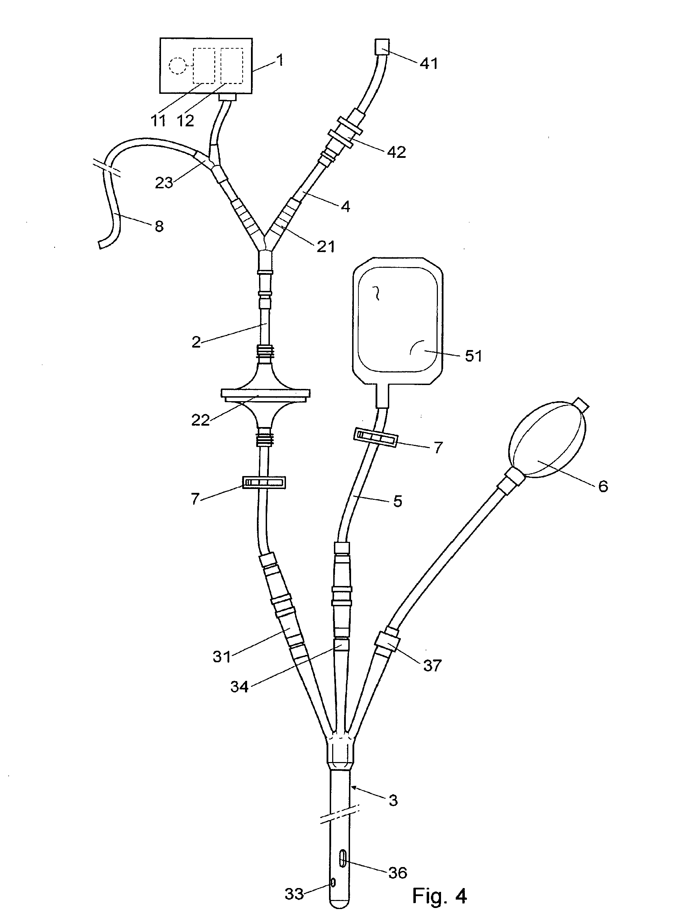

In one embodiment, represented in figure 4, the first duct (2) presents in the section between the by-pass (21) connecting the second duct (4) and the end connected to the insufflation apparatus (1), a second by-pass (23) to which a catheter (8) for the introduction of gas in a cavity of the patient through the mouth or the nose is connected.

Once the nature of the invention as well as an example of preferred embodiment have been sufficiently described, it is stated for all pertinent purposes that the materials, form, size and arrangement of the elements described are susceptible to changes, provided these do not involve an alteration of the essential characteristics of the invention that are claimed subsequently.

## Claims

1. Device for insufflating gas and collecting effluents from the body cavities of an individual, the device comprising: a gas insufflation apparatus (1), a first duct (2) for supplying the gas fed by the insufflation apparatus (1) toward the interior of a body cavity of the individual barriers (22) inserted in the first duct (2) to prevent the passage of effluents from the individual into the insufflation apparatus (1), a reservoir for (51) for collecting said effluents, a member (3) that can be inserted in the body cavity of the individual, attachable to the first duct (2) and provided with an expandable annular element (30) for sealing the cavity perimeter of the individual; and inflation means (6) for inflating the expandable annular element (30) when the insertable member (3) is introduced into the body cavity of the individual; **characterized in that** the first duct (2) has a by-pass (21) which is connected to a second gas discharge duct (4), said second gas discharge duct (4) being provided with a safety valve (41) for the automatic discharge of gas to the outside when the pressure of the gas contained in the first duct (2) exceeds a predetermined value, said by-pass (21) being in the section between the insufflation apparatus (1) and the barriers (22) that block the passage of the effluents into the insufflation apparatus (1) ; and **in that** the insertable member (3) comprises, at its rear end:
- a first opening (31) for connecting the first duct (2) with a first inner channel (32) of the insertable member (3), said first inner channel (32) being provided with an outlet opening (33) arranged in an area next to the front end of the insertable member (3);
- a second opening (34) for connecting an effluent discharge duct (5) with a second inner channel (35) of the insertable member (3), said second inner channel (35) being provided with an outlet opening (36) arranged in an area next to the front end of the insertable member (3); said effluent discharge duct (5) being connected by its rear end to the effluent collecting reservoir (51); and
- a third opening (37) for connecting the inflation means (6) of the expandable annular element (30) with a third inner channel (38) of the insertable member (3), said third inner channel (38) being provided with an outlet opening (39) situated inside the expandable annular element (30).

2. Device, according to claim 1, **characterized in that** the first duct (2) presents in the section between the by-pass (21) connecting the second gas discharge duct (4) and the end connected to the insufflation apparatus (1), a second by-pass (23) to which a catheter (8) for the introduction of gas in a cavity of the patient through the mouth or the nose is connected.

3. Device, according to any of the previous claims, **characterized in that** the second gas discharge duct (4) comprises scented barriers (42).

4. Device, according to any of the previous claims, **characterized in that** the gas insufflation apparatus (1) comprises means (11) for warming the gas to be supplied.

5. Device, according to claim 4, **characterized in that** the gas warming means (11) comprise a thermostat (12) that regulates the warming of the gas to a temperature of about 37.5 Celsius degrees.

6. Device, according to any of the previous claims, **characterized in that** at least one of the barriers (22) arranged in the first duct (2) comprises antiviral or antibacterial materials.

7. Device, according to any of the previous claims, **characterized in that** at least one of the barriers (22) comprises a hydrophobic filter.

8. Device, according to any of the previous claims, **characterized in that** it comprises clamping means, clamps (7) or a lock arranged in the first duct (2) before the barriers (22) and in the effluent discharge duct (5).

## Patentansprüche

1. Vorrichtung zum Einblasen von Gas und zur Sammlung von Effluenzien aus dem Körper eines Patienten, wobei die Vorrichtung folgendes umfasst: ein Gaseinblasgerät (1), einen ersten Kanal (2) für die Zufuhr von Gas, das vom Einblasgerät (1) in das Innere einer Körperöffnung eines Patienten zugeführt wird, im ersten Kanal (2) eingefügte Sperren (22), die den Durchfluss von Effluenzien vom Patienten in das Einblasgerät (1) verhindern, ein Behälter (51) zur Sammlung der Effluenzien, ein Element (3), das in die Körperöffnung eines Patienten eingeführt und am ersten Kanal (2) befestigt werden kann, und das mit einem erweiterbaren ringförmigen Element (30) zur Abdichtung der Öffnung des Patienten ausgestattet ist, und ein Aufblasinstrument (6) zum Aufblasen des erweiterbaren ringförmigen Elements (30), wenn das einführbare Element (3) in die Körperöffnung des Patienten eingeführt wird; **gekennzeichnet dadurch, dass** der erste Kanal (2) einen Bypass (21) hat, der mit einem zweiten Gasaustrittskanal (4) verbunden ist, wobei der zweite Gasaustrittskanal (4) versehen ist mit einem Sicherheitsventil (41) zum automatischen Ausblasen des Gases nach Außen, wenn der Druck des Gases im ersten Kanal (2) einen vorgegebenen Wert übersteigt, wobei sich der Bypass (21) im Bereich zwischen dem Einblasgerät (1) und den Sperren (22), die den Durchfluss der Effluenzien in das Einblasgerät (1) blockieren, befindet; und dadurch, dass das einführbare Element (3) an seinem hinteren Ende folgendes umfasst:
- eine erste Öffnung (31) zum Anschluss des ersten Kanals (2) an eine erste innere Röhre (32) des einführbaren Elements (3), wobei die erste innere Röhre (32) mit einer Auslassöffnung (33) ausgestattet ist, die in einem Bereich im Anschluss an das vordere Ende des einführbaren Elements (3) angeordnet ist;
- eine zweite Öffnung (34) zum Anschluss eines Effluenzienaustrittskanals (5) an eine zweite innere Röhre (35) des einführbaren Elements (3), wobei die zweite innere Röhre (35) mit einer Auslassöffnung (36) ausgestattet ist, die in einem Bereich im Anschluss an das vordere Ende des einführbaren Elements (3) angeordnet ist, wobei der Effluenzienaustrittskanal (5) mit seinem hinteren Ende an den Effluenziensammelbehälter (51) angeschlossen ist; und
- eine dritte Öffnung (37) zum Anschluss des Aufblasinstruments (6) des erweiterbaren ringförmigen Elements (30) an eine dritte innere Röhre (38) des einführbaren Elements (3), wobei die dritte innere Röhre (38) mit einer Auslassöffnung (39) ausgestattet ist, die sich innerhalb des erweiterbaren ringförmigen Elements (30) befindet.

2. Vorrichtung nach Anspruch 1, **dadurch gekennzeichnet, dass** der erste Kanal (2) im Abschnitt zwischen dem Bypass (21), der den zweiten Gasaustrittskanal (4) und das mit dem Einblasgerät (1) verbundene Ende verbindet, einen zweiten Bypass (23) hat, an dem ein Katheter (8) für die Einführung von Gas in die Öffnung des Patienten über den Mund oder die Nase angeschlossen ist.

3. Vorrichtung nach einem der vorhergehenden Ansprüche, **dadurch gekennzeichnet, dass** der zweite Gasaustrittskanal (4) Duftsperren (42) umfasst.

4. Vorrichtung nach einem der vorhergehenden Ansprüche, **dadurch gekennzeichnet, dass** das Gaseinblasgerät (1) ein Instrument (11) zum Erhitzen des zuzuführenden Gases umfasst.

5. Vorrichtung nach Anspruch 4, **dadurch gekennzeichnet, dass** das Gaserhitzungsinstrument (11) ein Thermostat (12) umfasst, das die Erhitzung des Gases auf eine Temperatur von ca. 37,5 Grad Celsius reguliert.

6. Vorrichtung nach einem der vorhergehenden Ansprüche, **dadurch gekennzeichnet, dass** mindestens eine der im ersten Kanal (2) angeordneten Sperren (22) antivirales oder antibakterielles Material umfasst.

7. Vorrichtung nach einem der vorhergehenden Ansprüche, **dadurch gekennzeichnet, dass** mindestens eine der Sperren (22) einen hydrophoben Filter umfasst.

8. Vorrichtung nach einem der vorhergehenden Ansprüche, **dadurch gekennzeichnet, dass** sie Spannmittel, Halterungen (7) oder einen Verschluss umfasst, die im ersten Kanal (2) vor den Sperren (22) und im Effluenzienaustrittskanal (5) angeordnet sind.

## Revendications

1. Dispositif d'insufflation de gaz et de collecte d'effluents à partir des cavités corporelles d'un individu, le dispositif comprenant: un appareil d'insufflation de gaz (1), un premier conduit (2) servant à acheminer le gaz conduit par l'appareil d'insufflation (1) vers l'intérieur d'une cavité corporelle de l'individu, des barrières (22) introduites dans le premier conduit (2) pour empêcher le passage d'effluents issus de l'individu dans l'appareil d'insufflation (1), un réservoir (51) servant à collecter lesdits effluents, un élément (3) qu'on peut insérer dans la cavité corporelle de l'individu, qu'on peut attacher au premier conduit (2) et doté d'un élément annulaire extensible (30) pour étancher le périmètre de la cavité de l'individu, et un moyen de gonflage (6) servant à gonfler l'élément annulaire extensible (30) lorsqu'on introduit l'élément insérable (3) dans la cavité corporelle de l'individu; **caractérisé en ce que** le premier conduit (2) comporte une dérivation (21) qui est raccordée à un deuxième conduit d'évacuation de gaz (4), ledit deuxième conduit d'évacuation de gaz (4) étant doté d'une soupape de sécurité (41) pour l'évacuation automatique de gaz vers l'extérieur lorsque la pression du gaz contenu dans le premier conduit (2) dépasse une valeur prédéfinie ; ladite dérivation (21) se trouvant dans la section située entre l'appareil d'insufflation (1) et les barrières (22) qui bloquent le passage des effluents dans l'appareil d'insufflation (1) et **en ce que** l'élément insérable (3) comprend, au niveau de son extrémité arrière:
- une première ouverture (31) servant à raccorder le premier conduit (2) à un premier canal intérieur (32) de l'élément insérable (3), ledit premier canal intérieur (32) étant doté d'une ouverture de sortie (33) disposée dans une zone proche de l'extrémité avant de l'élément insérable (3);
- une deuxième ouverture (34) servant à raccorder un conduit d'évacuation d'effluents (5) avec un deuxième canal intérieur (35) de l'élément insérable (3), ledit deuxième canal intérieur (35) étant doté d'une ouverture de sortie (36) disposée dans une zone proche de l'extrémité avant de l'élément insérable (3), ledit conduit d'évacuation d'effluents (5) étant raccordé par son extrémité arrière au réservoir (51) de collecte desdits effluents; et
- une troisième ouverture (37) servant à raccorder le moyen de gonflage (6) de l'élément annulaire extensible (30) avec un troisième canal intérieur (38) de l'élément insérable (3), ledit troisième canal intérieur (38) étant doté d'une ouverture de sortie (39) située à l'intérieur de l'élément annulaire extensible (30).

2. Dispositif selon la revendication 1, **caractérisé en ce que** le premier conduit (2) présente, dans la section entre la dérivation (21) raccordant le deuxième conduit d'évacuation de gaz (4) et l'extrémité raccordée à l'appareil d'insufflation (1), une deuxième dérivation (23) à laquelle un cathéter (8) destiné à l'introduction de gaz dans une cavité du patient à travers la bouche ou le nez est raccordé.

3. Dispositif selon l'une quelconque des revendications précédentes, **caractérisé en ce que** le deuxième conduit d'évacuation de gaz (4) comprend des barrières parfumées (42).

4. Dispositif selon l'une quelconque des revendications précédentes, **caractérisé en ce que** l'appareil d'insufflation de gaz (1) comprend un moyen (11) servant à réchauffer le gaz à fournir.

5. Dispositif selon la revendication 4, **caractérisé en ce que** les moyens de réchauffement du gaz (11) comprennent un thermostat (12) qui régule le réchauffement du gaz à une température d'environ 37,5°C.

6. Dispositif selon l'une quelconque des revendications précédentes, **caractérisé en ce qu'au** moins une des barrières (22) disposée dans le premier conduit (2) comprend des matériaux antiviraux et antibactériens.

7. Dispositif selon l'une quelconque des revendications précédentes, **caractérisé en ce qu**'au moins une des barrières (22) comprend un filtre hydrophobe.

8. Dispositif selon l'une quelconque des revendications précédentes, **caractérisé en ce qu'**il comprend des moyens de serrage, des pinces (7) ou un verrou disposé dans le premier conduit (2) avant les barrières (22) et dans le conduit d'évacuation des effluents (5).
